# EUROPEAN PATENT SPECIFICATION

(11) **EP 1 374 771 B1**
(45) Date of publication and mention of the grant of the patent: **08.03.2006**
(21) Application number: 03014234.3
(22) Date of filing: 25.06.2003
(51) Int. Cl.: A61B 5/15

(54) **Polygonal cross section lancet needle**
Polygonale Lanzettennadel
Aiguille polygonale pour lancette

(30) Priority: 25.06.2002 US 390893 P
(43) Date of publication of application: 02.01.2004
(73) Proprietor: Bayer HealthCare LLC, Tarrytown, New York 10591 (US)
(72) Inventor: Brenneman, Allen J., Goshen, Indiana 46526 (US); Kheiri, Mohammad A., Elkhart, Indiana 46514 (US)
(74) Representative: Linhart, Angela

(56) References cited:
- EP-A- 1 074 219
- WO-A-00/74763
- US-A- 4 414 975

## Description

### FIELD OF THE INVENTION

The present invention relates generally to a lancet needle for lancing fingers and other body sites to obtain small samples of blood or interstitial fluid for diagnostic testing. More particularly, the invention relates to a lancet needle having a polygonal or multisided cross section.

### BACKGROUND OF THE INVENTION

People with certain medical conditions, such as diabetics, often have to perform diagnostic tests on their blood for glucose and other analytes several times a day. To perform these tests, a site on the body is selected and lanced using a lancet assembly that includes a lancet needle. These lancet needles are made from round stainless steel wires with tips that are ground to form two or three facets on the tips. Round needles force the skin open after initial penetration and only cut at the ground facets which results in a large puncture that is painful and requires a long time to heal.

Recently, instruments for performing diagnostic tests have been developed that require much smaller samples of blood or interstitial fluid than the sample size required by earlier instruments. In addition, patients have demanded less painful lancing methods. Thus, there is a need for lancing needles whose puncture is less painful than current needles and produce a small wound that is quick to heal.

### SUMMARY OF THE INVENTION

The present invention is directed to a multisided lancet needle that makes a small, quick healing cut at a puncture site. The lancet needle is defined by a body having a polygonal cross section and a first end and a second end. The first end is ground at one side at an angle, for example, an angle between about 5° and about 10°, to form a sharp edge for lancing the skin of a user. The cross section of the needle body can be square, triangular, rectangular, or any similar multisided configuration.

### BRIEF DESCRIPTION OF THE FIGURES

Other objects and advantages of the invention will become apparent upon reading the following detailed description and upon reference to the drawings in which:
FIG. 1 is a perspective view of a square lancet needle with a single cut;
FIG. 2 is a perspective view of a square lancet needle with a double cut, which is prior art;
FIG. 3 is a perspective view of a square lancet needle with a diagonal cut;
FIG. 4 is a perspective view of a triangular lancet needle with a single cut;
FIG 5 is a perspective view of a rectangular lancet needle with a single cut;
FIG 6 is a plan view of a round wire with one end ground to a preferred configuration;
FIG. 7 is a view of the wire illustrated in FIG. 6 with the one end ground to define a sharp edge; and
FIG. 8 is an enlarged view of a square lancet needle with a tip ground at an angle.

### DETAILED DESCRIPTION OF THE ILLUSTRATED EMBODIMENTS

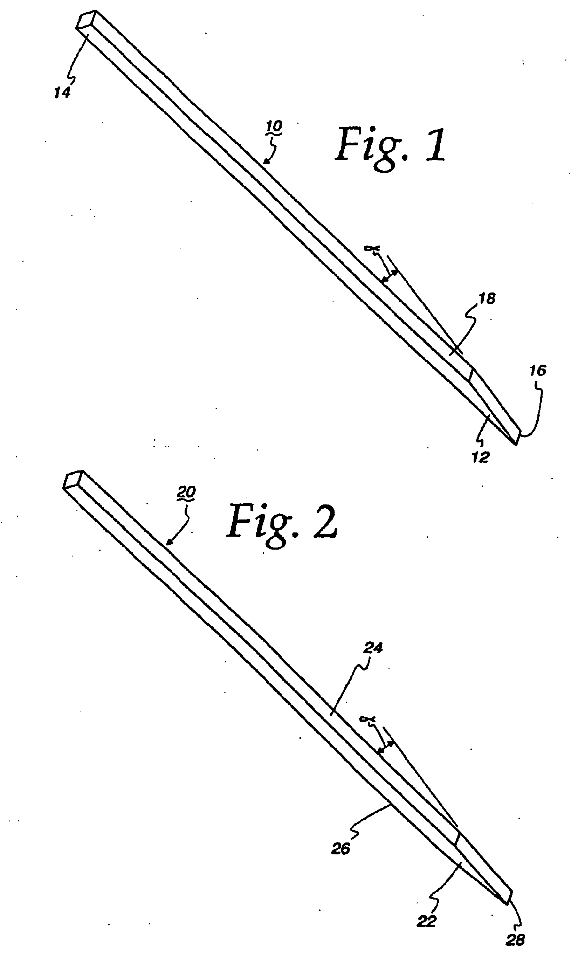

Referring to FIG. 1, there is illustrated a lancet needle 10 having a square cross section. The lancet needle 10 is formed of stainless steel but other material may be used. The lancet needle 10 has a first end 12 and a second end 14. The first end 12 has a sharp edge or tip 16 formed by grinding one side 18 of the lancet needle 10 on an angle V to the one side 18. Preferably, the angle V is in the range of between about 5° to about 10° but other angles may be used. It has been learned that this angle V in the range of about 5° to about 10° provides a sufficiently sharp edge or tip 16 to penetrate and cut human skin and tissue in a very clean way with less pain to the user than the pain experienced using prior art round needles.

Another lancet needle 20 of square cross sectional configuration is shown in FIG. 2. This lancet needle 20 is known from US 4,414975 and forms the preamble of claims 1 and 10. It differs from the claimed needles in that its first end 22 is ground on opposite sides 24, 26 to form a sharp edge or tip 28.

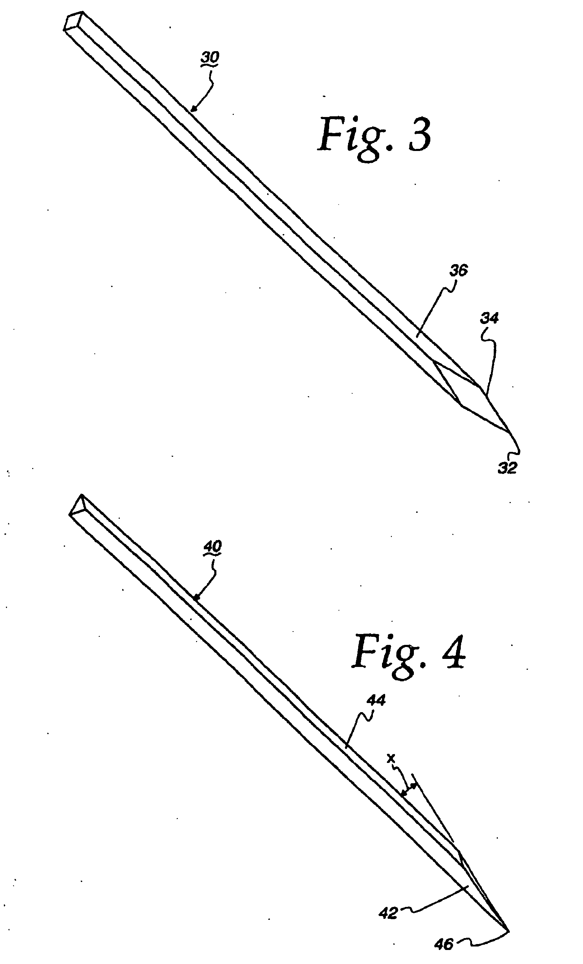

A lancet needle 30 of square cross sectional configuration is illustrated in FIG. 3. The lancet needle 30 is ground on a diagonal by a diagonal cut to form a sharp edge or tip 32 on a first end 34 of the lancet needle 30. The first end 34 is ground diagonally 45° from a first side 36 at an angle in the range of about 5° to about 10° although other angles may be used.

A lancet needle 40 with a triangular cross sectional configuration is shown in FIG. 4. A first end 42 of the lancet needle 40 is ground on a side 44 at an angle X in the range of between about 5° and about 10° to form a sharp chisel point or edge 46.

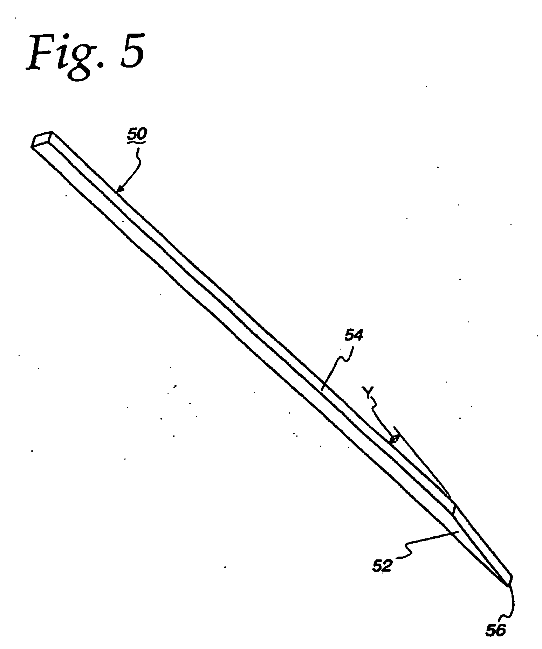

In FIG. 5 there is illustrated a lancet needle 50 having a rectangular cross sectional configuration. A first end 52 of the lancet needle 50 has one side 54 ground at angle Y that is in the range of between about 5° to about 10° to define a sharp edge 56 for piercing skin and tissue.

The sharp edges or tips 16, 28, 32, 46, 56 of the needles 10, 20, 30, 40, 50 penetrate a user's skin and continue to cut the skin and tissue in a clean way resulting in a lower pain level and a cut or puncture that heals faster than a cut from a conventional round lancet needle. This is because the multisided cross sections cut on all sides and do not force skin open as much as round needles. Thus, multisided configurations such as hexagonal are also contemplated.

Manufacturing these lancet needles 10, 20, 30, 40, 50 is similar to the manufacture of conventional round needles. Multisided cross sectional wires are extruded from a round stainless steel wire and cut into short workable lengths for grinding. The tips or first ends of a large number of these wires are ground to the desired angle at the same time. The needles are then cleaned and deburred before being molded in plastic to form a lancet. The molded lancets are then sterilized by radiation.

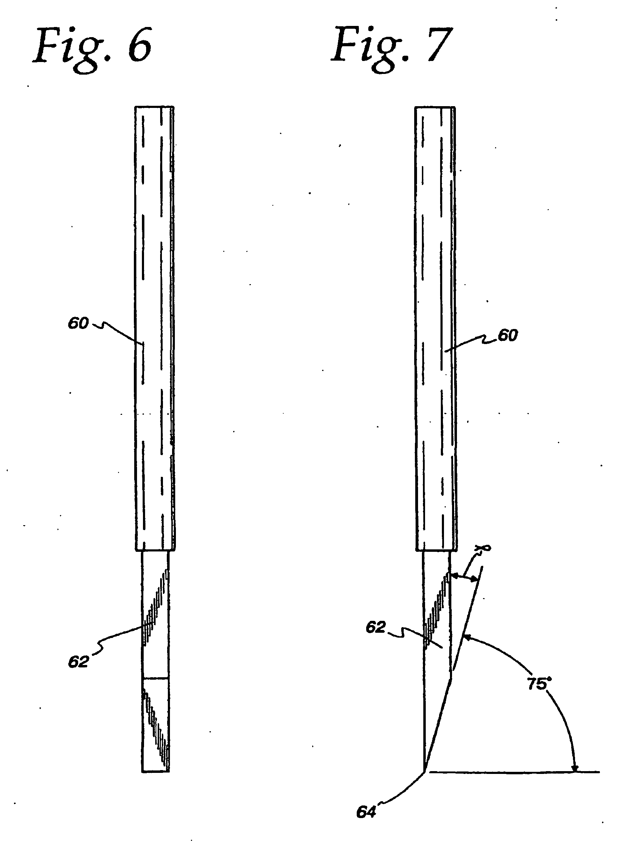

An alternative method of manufacture can be envisioned which will require less change to the manufacturing of conventional round needles. Rather than extruding round wire to a different cross-section, a conventional round wire 60 is ground on a first end 62 to a preferred multisided cross section (e.g., square, triangular, rectangular, etc.) (FIG. 6). The first end 62 is then ground at an angle from about 5° to about 10° to form a sharp edge or tip 64 (FIG. 7). At this point the ground wire 60 can be insert molded into plastic. This method allows the use of large diameter round wires 60, 22-26 gauge for example, since the first end 62 can be ground to a small cross section profile (29-32 gauge for example). The larger gauge is easier to handle for manufacturing and has more surface area to lock into molded plastic. This results in the same cutting edges as in the previously described lancets. In addition the step formed at the transition from the round to the smaller cross section will also provide a means of locking the needle into the plastic.

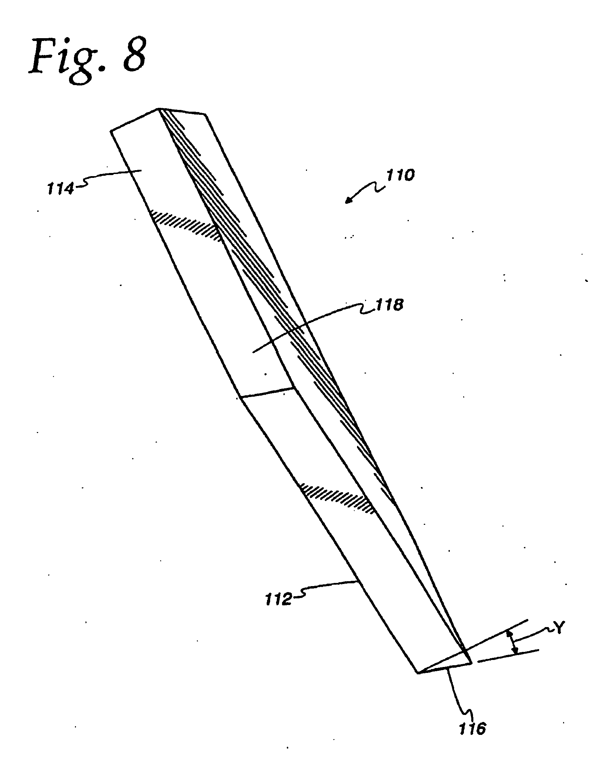

It has been found that grinding an end of a needle at an angle to the longitudinal axis of the needle results in an angle at the tip which can start a cut in skin at an end of the tip rather than a cut being started along the entire length of the tip. This causes less pain to the user or patient. This angled grind is shown in FIG. 8. A lancet needle 110 may be square or rectangular with a first end 112 and a second end 114. The needle 110 can be ground on one side 118 or a double grind as in FIG. 2 but the grind in FIG. 8 is at an angle to the longitudinal axis of the needle 110 resulting in an angled tip 116 at an angle Y in the range of 0° to 15°.

## Claims

1. A needle (10) for a lancet assembly, comprising:
a needle body (10), said needle body being of a multiside configuration including a first end (12) and a second end (14), **characterised in that** only one side of said needle body at said first end (12) being at an angle to the longitudinal axis of the needle to define a sharp distal edge or tip (16).

2. The needle claimed in claim 1 wherein the cross section of said needle body is square.

3. The needle claimed in claim 1 wherein the cross section of said needle body is triangular.

4. The needle claimed in claim 1 wherein said portion of said first end is at an angle between about 5° and about 10°.

5. The needle claimed in claim 1 wherein the cross section of said needle body is rectangular.

6. The needle claimed in claim 1 wherein said body is square, one side of said square being at an angle between about 5° to about 10° to define said sharp edge or tip.

7. The needle claimed in claim 1 wherein said body is in the configuration of a triangle with said one portion of said triangle being at an angle of between about 5° and about 10° to define said sharp edge or tip

8. The needle claimed in claim 1 wherein said sharp edge or tip being at an angle to the longitudinal axis of said multisided body.

9. The needle claimed in claim 8 wherein said angle is between 0° and 15°.

10. A method of making a multisided lancet needle, comprising:
providing a multisided body (10) having a first end (12) and a second end (14), and **characterised in that** only one angle is formed relative to the longitudinal axis of the needle in said first end to define a distal sharp edge or tip (16).

11. The method claimed in claim 10 wherein said step of providing a multisided body comprises providing a four sided body.

12. The method claimed in claim 10 wherein said step of providing a multisided body comprises providing a three sided body.

13. The method claimed in claim 10 wherein said step of forming an angle comprises forming an angle between from about 5° to about 10°.

14. The method claimed in claim 10 wherein said step of providing a multisided body comprises providing a round wire having a first end,
grinding at least a portion of said first end into a multisided configuration, and
grinding said first end at an angle from about 5° to about 10° to form a sharp edge.

## Patentansprüche

1. Nadel (10) für eine Lanzettenanordnung, umfassend:
einen Nadelkörper (10), welcher Nadelkörper eine mehrseitige Konfiguration aufweist, die ein erstes Ende (12) und ein zweites Ende (14) umfasst, **dadurch gekennzeichnet, dass** nur eine Seite des Nadelkörpers am ersten Ende (12) in einem solchen Winkel zur Längsachse der Nadel steht, dass eine scharfe distale Kante oder Spitze (16) definiert ist.

2. Nadel nach Anspruch 1, worin der Querschnitt des Nadelkörpers quadratisch ist.

3. Nadel nach Anspruch 1, worin der Querschnitt der Nadel dreieckig ist.

4. Nadel nach Anspruch 1, worin der Abschnitt des ersten Endes einen Winkel zwischen etwa 5° und etwa 10° aufweist.

5. Nadel nach Anspruch 1, worin der Querschnitt des Nadelkörpers rechteckig ist.

6. Nadel nach Anspruch 1, worin der Körper quadratisch ist, wobei eine Seite des Quadrats einen Winkel zwischen etwa 5° und etwa 10° aufweist, um die scharfe Kante oder Spitze zu definieren.

7. Nadel nach Anspruch 1, worin der Körper die Konfiguration eines Dreiecks aufweist, wobei der eine Abschnitt des Dreiecks einen Winkel zwischen etwa 5° und etwa 10° aufweist, um die scharfe Kante oder Spitze zu definieren.

8. Nadel nach Anspruch 1, worin die scharfe Kante oder Spitze in einem Winkel zur Längsachse des mehrseitigen Körpers steht.

9. Nadel nach Anspruch 8, worin der Winkel zwischen 0° und 15° liegt.

10. Verfahren zur Herstellung einer mehrseitigen Lanzettennadel, umfassend:
das Bereitstellen eines mehrseitigen Körpers (10) mit einem ersten Ende (12) und einem zweiten Ende (14), **dadurch gekennzeichnet, dass** an dem ersten Ende nur ein Winkel in Bezug auf die Längsachse der Nadel ausgebildet wird, um eine distale scharfe Kante oder Spitze (16) zu definieren.

11. Verfahren nach Anspruch 10, worin der Schritt der Bereitstellung eines mehrseitigen Körpers die Bereitstellung eines vierseitigen Körpers umfasst.

12. Verfahren nach Anspruch 10, worin der Schritt der Bereitstellung eines mehrseitigen Körpers die Bereitstellung eines dreiseitigen Körpers umfasst.

13. Verfahren nach Anspruch 10, worin der Schritt der Ausbildung eines Winkels die Ausbildung eines Winkels zwischen etwa 5° und etwa 10° umfasst.

14. Verfahren nach Anspruch 10, worin der Schritt der Bereitstellung eines mehrseitigen Körpers das Bereitstellen eines runden Drahts mit einem ersten Ende,
das Schleifen zumindest eines Abschnitts des ersten Endes zu einer mehrseitigen Konfiguration und
das Schleifen des ersten Endes in einem Winkel von etwa 5° bis etwa 10°, um eine scharfe Kante zu bilden, umfasst.

## Revendications

1. Aiguille (10) pour agencement de lancette, comprenant :
un corps d'aiguille (20), ledit corps d'aiguille étant d'une configuration à côtés multiples comprenant une première extrémité (12) et une deuxième extrémité (14), **caractérisé en ce que** seul un côté dudit corps d'aiguille à ladite première extrémité (12) est à un angle par rapport à l'axe longitudinal de l'aiguille pour définir une arête vive, ou pointe, distale (16).

2. Aiguille selon la revendication 1, dans laquelle la section transversale dudit corps d'aiguille est carrée.

3. Aiguille selon la revendication 1, dans laquelle la section transversale dudit corps d'aiguille est triangulaire.

4. Aiguille selon la revendication 1, dans laquelle ladite portion de ladite première extrémité est à un angle compris entre environ 5° et environ 10°.

5. Aiguille selon la revendication 1, dans laquelle la section transversale dudit corps d'aiguille est rectangulaire.

6. Aiguille selon la revendication 1, dans laquelle ledit corps est carré, un côté dudit carré étant à un angle compris entre environ 5° et environ 10° pour définir ladite arête vive, ou pointe.

7. Aiguille selon la revendication 1, dans laquelle ledit corps a la configuration d'un triangle, une dite portion dudit triangle étant à un angle compris entre environ 5° et environ 10° pour définir ladite arête vive, ou pointe.

8. Aiguille selon la revendication 1, dans laquelle ladite arête vive, ou pointe est à un angle par rapport à l'axe longitudinal dudit corps à côtés multiples.

9. Aiguille selon la revendication 8, dans laquelle ledit angle est compris entre 0° et 15°.

10. Procédé pour fabriquer une aiguille lancette à côtés multiples, comprenant:
la fourniture d'un corps à côtés multiples (10) comportant une première extrémité (12) et une deuxième extrémité (14), et **caractérisé en ce que** seul un angle est formé par rapport à l'axe longitudinal de l'aiguille dans ladite première extrémité pour définir une arête vive, ou pointe, distale (16).

11. Procédé selon la revendication 10, dans lequel ladite étape de fourniture d'un corps à côtés multiples comprend la fourniture d'un corps à quatre côtés.

12. Procédé selon la revendication 10, dans lequel ladite étape de fourniture d'un corps à côtés multiples comprend la fourniture d'un corps à trois côtés.

13. Procédé selon la revendication 10, dans lequel ladite étape de formation d'un angle comprend la formation d'un angle compris entre environ 5° et environ 10°.

14. Procédé selon la revendication 10, dans lequel ladite étape de fourniture d'un corps à côtés multiples comprend la fourniture d'un fil rond comportant une première extrémité,
meulage d'au moins une portion de ladite première extrémité en une configuration à côtés multiples, et
meulage de ladite première extrémité à un angle compris entre environ 5° et environ 10° pour former une arête vive.
